# EUROPEAN PATENT SPECIFICATION

(11) **EP 3 362 095 B1**
(45) Date of publication and mention of the grant of the patent: **25.11.2020**
(21) Application number: 16790279.0
(22) Date of filing: 12.10.2016
(51) Int. Cl.: A61K 45/06, A61K 31/585, A61P 27/02

(54) **METHODS AND PHARMACEUTICAL COMPOSITIONS FOR THE TREATMENT OF CHOROIDAL NEOVASCULARISATION**
VERFAHREN UND PHARMAZEUTISCHE ZUSAMMENSETZUNGEN ZUR BEHANDLUNG VON CHOROIDALER NEOVASKULARISATION
MÉTHODES ET PRÉPARATIONS PHARMACEUTIQUES POUR LE TRAITEMENT DE LA NEOVASCULARISATION CHOROIDIENNE

(30) Priority: 13.10.2015 EP 15306617
(43) Date of publication of application: 22.08.2018
(73) Proprietor: INSERM (Institut National de la Santé et de la Recherche Médicale), 75013 Paris Cédex 13 (FR); Université Paris Descartes, 75006 Paris (FR); Sorbonne Université, 75006 Paris (FR); Université Paris Diderot - Paris 7, 75013 Paris (FR); Fondation Asile Des Aveugles, 1000 Lausanne (CH)
(72) Inventor: BEHAR-COHEN, Francine, 75006 Paris (FR); ZHAO, Min, 75006 Paris (FR)
(74) Representative: Inserm Transfert
(86) International application number: PCT/EP2016/074470
(87) International publication number: WO 2017/064121

(56) References cited:
- US-A1- 2003 158 162
- US-A1- 2013 131 024
- KLAUBER N ET AL: "NEW ACTIVITY OF SPIRONOLACTONE INHIBITION OF ANGIOGENESIS IN VITRO AND IN VIVO", CIRCULATION, LIPPINCOTT WILLIAMS & WILKINS, US, vol. 94, no. 10, 15 November 1996 (1996-11-15), pages 2566-2571, XP001025755, ISSN: 0009-7322
- WILKINSON-BERKA J L ET AL: "Identification of a Retinal Aldosterone System and the Protective Effects of Mineralocorticoid Receptor Antagonism on Retinal Vascular Pathology", CIRCULATION RESEARCH., vol. 104, no. 1, 26 November 2008 (2008-11-26), pages 124-133, XP055228149, US ISSN: 0009-7330, DOI: 10.1161/CIRCRESAHA.108.176008
- DARUICH ALEJANDRA ET AL: "Central serous chorioretinopathy: Recent findings and new physiopathology hypothesis", PROGRESS IN RETINAL AND EYE RESEARCH, OXFORD, GB, vol. 48, 27 May 2015 (2015-05-27), pages 82-118, XP029258723, ISSN: 1350-9462, DOI: 10.1016/J.PRETEYERES.2015.05.003

## Description

### FIELD OF THE INVENTION:

The present invention relates to methods and pharmaceutical compositions for the treatment of choroidal neovascularisation.

### BACKGROUND OF THE INVENTION:

Choroidal neovascularization (CNV) may cause visual loss due to exudation of intraretinal or subretinal fluid, hemorrhage, or fibrosis at the macula. The most important causes of CNV are age-related macular degeneration (AMD) and pathological myopia (PM). Other causes such as inflammation, polypoidalchoroidopathy, or central serous chorioretinopathy are recognized. For instance AMD is the leading cause of blindness and severe visual impairment in all high-income countries that affects the elderly. The prevalence of the disease increases exponentially with every decade post age 50 years. PM is common in about 2% of the general population. Myopic CNV is a leading cause of severe visual loss and blindness in the eyes with PM, which develops in 4 - 11% of affected eyes. It is particularly prevalent among people under 50 years old in Asians. CNV is the most important sight-threatening factor, which commonly happens secondary to AMD and PM. It is believed that oxidative stress to the retinal pigment epithelium (RPE) cells leads to accumulation of extracellular debris thereupon drusen forms and results in dysfunction of RPE cells via affecting the permeability of Bruch's membrane to nutrients. VEGF is an important proangiogenic element that is usually produced by RPE and retinal photoreceptors. In CNV, the RPE boosts atypical neovascularization and the VEGF is the main growth factor responsible for development and progress of new vessels. The idea of inhibiting the activities of VEGF with anti-VEGF drugs has been investigated in the management of ocular neovascular disorders, particularly for the treatment of its consequences i.e. sub retinal and or intraretinal fluid. The current anti-VEGF treatments do not induce a total regression of the CNV requiring repeated injections to maintain vision. Moreover, evidence regarding anti-VEGF therapy resistance suggests a need for alternative medicines for the treatment of CNV.

### SUMMARY OF THE INVENTION:

The present invention relates to methods and pharmaceutical compositions for the treatment of choroidal neovascularisation. In particular, the present invention is defined by the claims.

### DETAILED DESCRIPTION OF THE INVENTION:

The present invention relates to a method of treating choroidal neovascularisation in a subject in need thereof comprising administering to the subject a therapeutically effective amount of a mineralocorticoid receptor antagonist.

As used herein, the term "choroidal neovascularization" or "CNV" has its general meaning in the art and refers to new blood vessel growth from the choroid that extends under the retinal pigment epithelium, or into subretinal space, or a combination of both.

As used herein, the term "treatment" or "treat" refer to both prophylactic or preventive treatment as well as curative or disease modifying treatment, including treatment of subject at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

Representative diseases involving CNV include age-related macular degeneration, myopic choroidal neovascularization, idiopathic choroidal neovascularization, polypoidal chorioretinopathy associated or not to central serous chorioretinoapthy and some inflammatory conditions, can also induce this pathological condition. In the present invention, diseases involving CNV are not limited to the diseases described above, and also include diseases involving CNV that are caused by other diseases that result in damage at the level of Bruch's membrane and retinal pigment epithelia and subsequent inflammatory choroidal neovascularization, such as uveitis posterior, traumatic choroidal rupture, angioid streaks, and ocular histoplasmosis syndrome.

In some embodiments, the method of the present invention is particularly suitable for the treatment of CNV secondary to age-related macular degeneration (AMD) (also called age-related maculopathy). AMD is a progressive disease of the eye and is associated with choroidal neovascularization. AMD causes progressive damage to the macula, the central and most vital area of the retina, resulting in gradual loss of central vision. It is the most common cause of irreversible loss of central vision in the elderly. It is equally common among men and women. There are two forms of AMD: dry (atrophic) and wet (neovascular or exudative) AMD. Ninety percent of those with macular degeneration have the dry type. However, 90% of the blindness caused by macular degeneration occurs in the 10% of people who have the wet form.

In some embodiments, the method of the present invention is particularly suitable for the treatment of CNV secondary to pathological myopia. Myopic choroidal neovascularization is indeed the most common disease causing visual impairment in persons with pathologic myopia. When vessels are newly generated in the macular area, pigmented fibrous scars, which result in scotoma in the center of vision, are often formed. Excessive myopia, which is common in the Japanese people, is caused by the abnormal extension of the antero-posterior length of the eye (the eye's axis). As a result, various myopia-specific eyeground lesions, such as CNV, are developed at the posterior pole of eyeground, which can cause visual disorders.

In some embodiments, the method of the present invention is particularly suitable for the treatment of idiopathic choroidal neovascularisation. Idiopathic choroidal neovascularization often develops in one eye in young women, and can be diagnosed when uveitis, injury, collagen disease, infection, and the like can be negated. Tiny newly generated vascular tissues, hemorrhages and exudative changes, such as edema, may be found under the retina. The involvement of inflammation has been suggested in this disease, since it eases after a few months of treatment with anti-inflammatory steroids.

In some embodiments, the method of the present invention is particularly suitable for the treatment of polypoidal choroidal vasculopathy (PCV). PCV is a form of choroidal vasculoathy classified as a specific form of choroidal neovascularization but which genetic favouring factors are different from classical AMD and which present as aneurysmal dilations and leaky branching vessels and which has a more important resistance to classical anti VEGF treatments. PCV can be associated or not to central serous chorioretinopathy.

As used herein the term "MR antagonist" has its general meaning in the art. The MR antagonistic of a compound may be determined using various methods as described in J, Souque A, Wurtz JM, Moras D, Rafestin-Oblin ME. MolEndocrinol. 2000 Aug;14(8):1210-21; Fagart J, Seguin C, Pinon GM, Rafestin-Oblin ME. MolPharmacol. 2005 May;67(5):1714-22 or Hellal-Levy C, Fagart J, Souque A, Wurtz JM, Moras D, Rafestin-Oblin ME. MolEndocrinol. 2000 Aug;14(8):1210-21. Typically, the transfection of the human mineralocorticoid receptor in COS cells together with a luciferase-expressing reporter gene allows to measure its transactivation activity in the presence of a candidate compound. In the context of the present invention, mineralocorticoid receptor antagonists are typically selective for the mineralocorticoid receptor as compared with the related receptors such as androgen receptor, estrogen receptors, glucocorticoid receptor, progesterone receptor, thyroid hormone receptors, peroxisome proliferator-activated receptors, retinoic acid receptor, farnesoid x receptor, pregnane x receptor, liver X receptor, vitamin D receptor, retinoid x receptor and the constitutive androstane receptor. By "selective" it is meant that the affinity of the antagonist for the mineralocorticoid receptor is at least 10-fold, typically 25-fold, more typically 100-fold, still typically 500-fold higher than the affinity for the related receptors. MR antagonists constitute a class of pharmacological compounds that are well known by the skilled artisan.

For example, the mineralocorticoid receptor antagonists according to the invention generally are spirolactone-type steroidal compounds. The term "spirolactone-type" is intended to characterize a structure comprising a lactone moiety attached to a steroid nucleus, typically at the steroid "D" ring, through a spiro bond configuration. A subclass of spirolactone-type mineralocorticoid receptor antagonist compounds consists of epoxy-steroidal mineralocorticoid receptor antagonist compounds such as eplerenone. Another subclass of spirolactone-type antagonist compounds consists of non-epoxy-steroidal mineralocorticoid receptor antagonist compounds such as spironolactone.

The epoxy-steroidal mineralocorticoid receptor antagonist compounds used in the method of the present invention generally have a steroidal nucleus substituted with an epoxy-type moiety. The term "epoxy-type" moiety is intended to embrace any moiety characterized in having an oxygen atom as a bridge between two carbon atoms.

The term "steroidal," as used in the phrase "epoxy-steroidal," denotes a nucleus provided by a cyclopenteno-phenanthrene moiety, having the conventional "A", "B", "C", and "D" rings. The epoxy-type moiety may be attached to the cyclopenteno-phenanthrene nucleus at any attachable or substitutable positions, that is, fused to one of the rings of the steroidal nucleus or the moiety may be substituted on a ring member of the ring system. The phrase "epoxy-steroidal" is intended to embrace a steroidal nucleus having one or a plurality of epoxy-type moieties attached thereto.

Epoxy-steroidal mineralocorticoid receptor antagonists suitable for use in the present methods include a family of compounds having an epoxy moiety fused to the "C" ring of the steroidal nucleus. Examples include 20-spiroxane compounds characterized by the presence of a 9α, 11α-substituted epoxy moiety, such as:
- Pregn-4-ene-7,21-dicarboxylic acid, 9,11-epoxy-17-hydroxy-3-oxo-,γ-lactone, methyl ester, (7α,11α,17β)
- Pregn-4-ene-7,21-dicarboxylic acid, 9,11-epoxy-17-hydroxy-3-oxo-,dimethyl ester, (7α,11α,17β)
- 3'H-cyclopropa[6,7]pregna-4,6-diene-21-carboxylic acid, 9,11-epoxy-6,7-dihydro-17-hydroxy-3-oxo-, γ-lactone, (6*β*,7*β*,11*α*,17*β*)
- Pregn-4-ene-7,21-dicarboxylic acid, 9,11-epoxy-17-hydroxy-3-oxo-,7-(1-methylethyl)ester, monopotassium salt, (7α,11α,17β)
- Pregn-4-ene-7,21-dicarboxylic acid, 9,11-epoxy-17-hydroxy-3-oxo-,7-methylethyl)ester, monopotassium salt, (7α,11α,17β)
- 3' H-cyclopropa[6,7]pregna-1,4,6-triene-21-carboxylic acid, 9,11-epoxy-6,7-dihydro-17-hydroxy-3-oxo-, γ-lactone(6*β*,7*β*,11*α*)
- 3' H-cyclopropa[6,7]pregna-4,6-diene-21-carboxylic acid, 9,11-epoxy-6,7-dihydro-17-hydroxy-3-oxo-, methyl ester, (6*β*,7*β*,11*α*,17*β*)
- 3' H-cyclopropa[6,7]pregna-4,6-diene-21-carboxylic acid, 9,11-epoxy-6,7-dihydro-17-hydroxy-3-oxo-, monopotassium salt, (6*β*,7*β*,11*α*,17*β*)
- 3' H-cyclopropa[6,7]pregna-1,4,6-triene-21-carboxylic acid, 9,11-epoxy-6,7-dihydro-17-hydroxy-3-oxo-, γ-lactone(6*β*,7*β*,11*α*,17*β*)
- Pregn-4-ene-7,21-dicarboxylic acid, 9,11-epoxy-17-hydroxy-3-oxo-, γ-lactone, ethyl ester, (7α,11a,17β)
- Pregn-4-ene-7,21-dicarboxylic acid, 9,11-epoxy-17-hydroxy-3-oxo-, γ-lactone, 1-methylethyl ester (7α,11α,17β)

A particular benefit of using epoxy-steroidal mineralocorticoid receptor antagonists, as exemplified by eplerenone, is the high selectivity of this group of mineralocorticoid receptor antagonists for the mineralocorticoid receptor. The superior selectivity of eplerenone results in a reduction in side effects that can be caused by mineralocorticoid receptor antagonists that exhibit non-selective binding to related receptors, such as androgen or progesterone receptors.

These epoxy steroids may be prepared by procedures described in Grob et al., U.S. Pat. No. 4,559,332. Additional processes for the preparation of 9, 11-epoxy steroidal compounds and their salts are disclosed in Ng et al., WO97/21720 and Ng et al., WO98/25948.

Of particular interest is the compound eplerenone ((Pregn-4-ene-7,21-dicarboxylic acid, 9,11-epoxy-17-hydroxy-3-oxo-,γ-lactone, methyl ester, (7α,11α,17β)) (CAS No. 107724-20-9), also known as epoxymexrenone. Eplerenone is a mineralocorticoid receptor antagonist and has a higher selectivity for mineralocorticoid receptors than does, for example, spironolactone. Selection of eplerenone as the mineralocorticoid receptor antagonist in the present method would be beneficial to reduce certain side-effects such as gynecomastia that occur with use of mineralocorticoid receptor antagonists having less specificity.

Non-epoxy-steroidal mineralocorticoid receptor antagonists suitable for use in the present methods include a family of spirolactone-type compounds defined by Formula I: 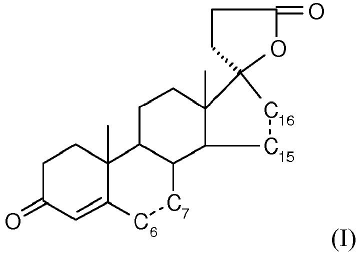 Wherein: 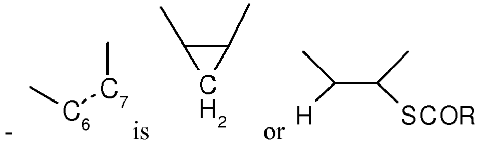
- R is lower alkyl of up to 5 carbon atoms, and

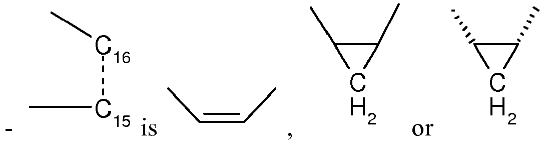

Lower alkyl residues include branched and unbranched groups, for example, methyl, ethyl and n-propyl.

Specific compounds of interest within Formula I are the following:
- 7*α*-acetylthio-3-oxo-4,15-androstadiene-[17(*β*-1')-spiro-5']perhydrofuran-2'-one;
- 3-oxo-7*α*-propionylthio-4,15-androstadiene-[17((*β*-1')-spiro-5']perhydrofuran-2'-one;
- 6*β*,7*β*-methylene-3-oxo4,15-androstadiene-[17((*β*-1')-spiro-5']perhydrofuran-2'-one;
- 15*α*,16*α*-methylene-3-oxo-4,7*α*-propionylthio-4-androstene[17(*β-*1')-spiro-5']perhydrofuran-2'-one;
- 6*β*,7*β*,15*α*,16*α*-dimethylene-3-oxo-4-androstene[17(*β*-1')-spiro-5']-perhydrofuran-2'-one;
- 7*α*-acetylthio-15*β*,16*β*-Methylene-3-oxo-4-androstene-[17(*β*-1')-spiro-5']perhydrofuran-2'-one;
- 15*β*,16*β*-methylene-3-oxo-7*β*-propionylthio-4-androstene-[17(*β*-1')-spiro-5']perhydrofuran-2'-one; and
- 6*β*,7*β*,15*β*,16*β*-dimethylene-3-oxo-4-androstene-[17(*β*-1')-spiro-5']perhydrofuran-2'-one.

Methods to make compounds of Formula I are described in U.S. Pat. No. 4,129,564 to Wiechart et al. issued on 12 Dec. 1978.

Another family of non-epoxy-steroidal compounds of interest is defined by Formula II: 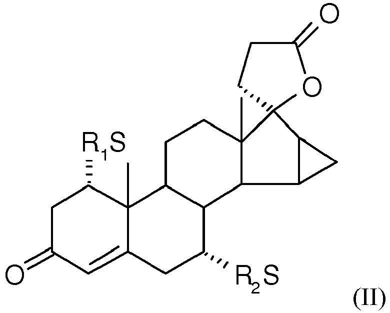 wherein R1 is C1-3-alkyl or C1-3 acyl and R2 is H or C1-3-alkyl.

Specific compounds of interest within Formula II are the following:
- 1α-acetylthio-15β,16β-methylene-7α-methylthio-3-oxo-17α-pregn-4-ene-21,17-carbolactone; and
- 15β,16β-methylene-1α,7α-dimethylthio-3-oxo-17α-pregn-4-ene-21,17-carbolactone.

Methods to make the compounds of Formula II are described in U.S. Pat. No. 4,789,668 to Nickisch et al. which issued 6 Dec. 1988.

Yet another family of non-epoxy-steroidal compounds of interest is defined by a structure of Formula III: 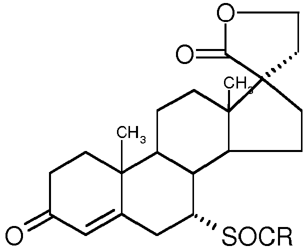 or 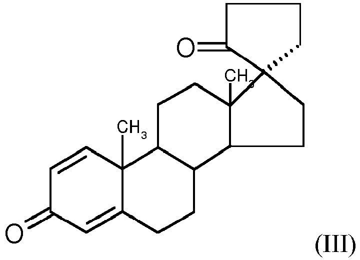 wherein R is lower alkyl, examples of which include lower alkyl groups of methyl, ethyl, propyl and butyl. Specific compounds of interest include:
- 3β,21-dihydroxy-17α-pregna-5,15-diene-17-carboxylic acid γ-lactone;
- 3β,21-dihydroxy-17a-pregna-5,15-diene-17-carboxylic acid γ-lactone 3-acetate;
- 3β,21-dihydroxy-17a-pregn-5-ene-17-carboxylic acid γ-lactone;
- 3β,21-dihydroxy-17α-pregn-5-ene-17-carboxylic acid γ-lactone 3-acetate;
- 21-hydroxy-3-oxo-17α-pregn-4-ene-17-carboxylic acid γ-lactone;
- 21-hydroxy-3-oxo-17α-pregna-4,6-diene-17-carboxylic acid γ-lactone;
- 21-hydroxy-3-oxo-17α-pregna-1,4-diene-17-carboxylic acid γ-lactone;
- 7α-acylthio-21-hydroxy-3-oxo-17α-pregn-4-ene-17-carboxylic acid γ-lactone; and
- 7α-acetylthio-21-hydroxy-3-oxo-17α-pregn-4-ene-17-carboxylic acid γ-lactone.

Methods to make the compounds of Formula III are described in U.S. Pat. No. 3,257,390 to Patchett which issued 21 Jun. 1966.

Still another family of non-epoxy-steroidal compounds of interest is represented by Formula IV: 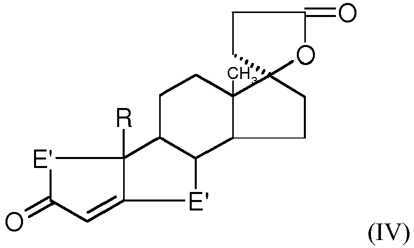 wherein E' is selected from the group consisting of ethylene, vinylene and (lower alkanoyl)thioethylene radicals, E" is selected from the group consisting of ethylene, vinylene, (lower alkanoyl)thioethylene and (lower alkanoyl)thiopropylene radicals; R is a methyl radical except when E' and E" are ethylene and (lower alkanoyl) thioethylene radicals, respectively, in which case R is selected from the group consisting of hydrogen and methyl radicals; and the selection of E' and E" is such that at least one (lower alkanoyl)thio radical is present.

One family of non-epoxy-steroidal compounds within Formula IV is represented by Formula V:

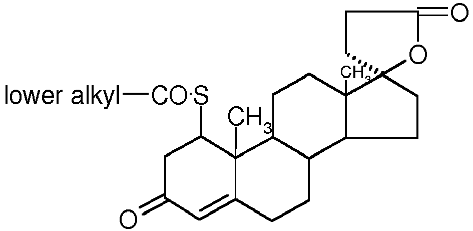

Another compound of Formula V is 1-acetylthio-17α-(2-carboxyethyl)-17β-hydroxy-androst-4-en-3-one lactone.

Another family of non-epoxy-steroidal compounds within Formula IV is represented by Formula VI:

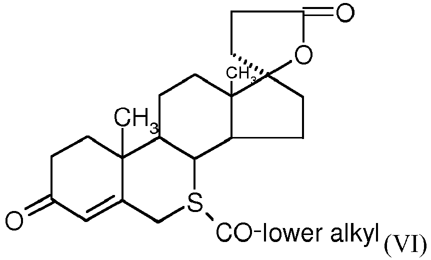

Exemplary compounds within Formula VI include the following:
- 7α-acetylthio-17α-(2-carboxyethyl)-17β-hydroxy-androst-4-en-3-one lactone;
- 7β-acetylthio-17α-(2-carboxyethyl)-17β-hydroxy-androst-4-en-3-one lactone;
- 1α,7α-diacetylthio-17α-(2-carboxyethyl)-17β-hydroxy-androsta-4,6-dien-3-one lactone;
- 7α-acetylthio-17αe-(2-carboxyethyl)-17β-hydroxy-androsta-1,4-dien-3-one lactone;
- 7α-acetylthio-17α-(2-carboxyethyl)-17β-hydroxy-19-norandrost-4-en-3-one lactone; and
- 7α-acetylthio-17α-(2-carboxyethyl)-17β-hydroxy-6α-methylandrost-4-en-3-one lactone.

In Formulae IV-VI, the term "alkyl" is intended to embrace linear and branched alkyl radicals containing one to about eight carbons. The term "(lower alkanoyl)thio" embraces radicals of the formula lower alkyl

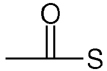

Of particular interest is the compound spironolactone (17-hydroxy-7α-mercapto-3-oxo-17α-pregn-4-ene-21-carboxylic acid γ-lactone acetate) having the following structure:

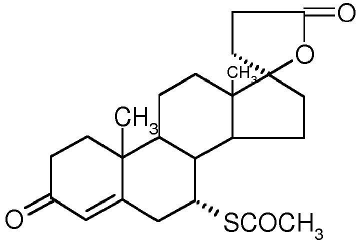

Methods to make compounds of Formulae IV-VI are described in U.S. Pat. No. 3,013,012 to Cella et al. which issued 12 Dec. 1961. Spironolactone is sold by G. D. Searle & Co., Skokie, Ill., under the trademark "ALDACTONE", in tablet dosage form at doses of 25 mg, 50 mg and 100 mg per tablet.

Another family of steroidal mineralocorticoid receptor antagonists is exemplified by drospirenone, (6R-(6*α*,7*α*,8*β*,9*α*,10*β*,13*β*,14*α,*15*α*,16*α*,17*β*))-1,3',4',6, 7,8,9,10,11,12,13,14,15,16,20,21-hexadecahydro-10, 13-dimethylspiro [17H-dicyclopropa(6,7:15,16)cyclopenta(a)phenanthrene-17,2'(5'H)-furan)-3,5'(2H)-dione, CAS registration number 67392-87-4. Methods to make and use drospirenone are described in patent GB 1550568 1979, priority DE 2652761 1976.

Crystalline forms that are easily handled, reproducible in form, easily prepared, stable, and which are non-hygroscopic have been identified for the mineralocorticoid receptor antagonist eplerenone. These include Form H, Form L, various crystalline solvates and amorphous eplerenone. These forms, methods to make these forms, and use of these forms in preparing compositions and medicaments, are disclosed in Barton et al., WO 01/41535 and Barton et al., WO 01/42272.

Mineralocorticoid receptor antagonists according to the invention may also be non-steroidal. For example, classes of non-steroidal MR antagonists have just begun to emerge over the past few years (Meyers, Marvin J1; Hu, Xiao Expert Opinion on Therapeutic Patents, Volume 17, Number 1, January 2007, pp. 17-23(7) and Piotrowski DW. Mineralocorticoid Receptor Antagonists for the Treatment of Hypertension and Diabetic NephropathyJ. Med. Chem. 2012, 55, 7957-7966). For instance, dihydropyrymidines have been shown to display MR antagonism (Activation of Mineralocorticoid Receptors by Exogenous Glucocorticoids and the Development of Cardiovascular Inflammatory Responses in Adrenalectomized Rats. Young MJ, Morgan J, Brolin K, Fuller PJ, Funder JW. Endocrinology. 2010 Apr 21). Furthermore, Arhancet el al. disclose other class of non-steroidal MR antagonists (Arhancet GB, Woodard SS, Dietz JD, Garland DJ, Wagner GM, Iyanar K, Collins JT, Blinn JR, Numann RE, Hu X, Huang HC. Stereochemical Requirements for the Mineralocorticoid Receptor Antagonist Activity of Dihydropyridines. J Med Chem. 2010 Apr 21). Other exemplary non-steroidal mineralocorticoid receptor antagonists include but are not limited to those described in US 20090163472 WO2004052847, WO 2008053300 WO2008104306, WO2007025604, WO201264631, WO2008126831, WO2012008435, WO2010104721, WO200985584, WO200978934, WO2008118319, WO200917190, WO200789034, WO2012022121, WO2012022120, WO2011141848 and WO200777961.

In some embodiments, the mineralocorticoid receptor antagonist is selected from the group consisting of:

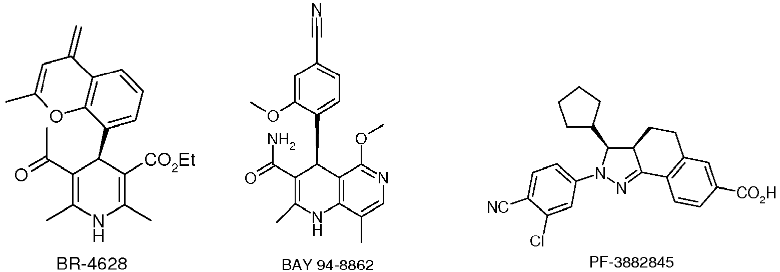

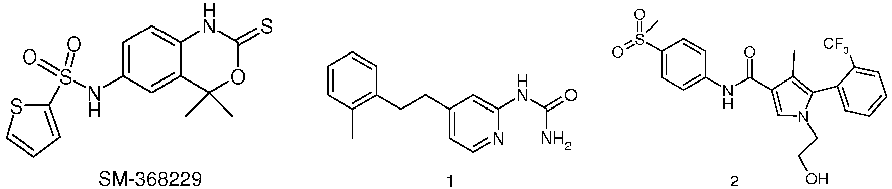

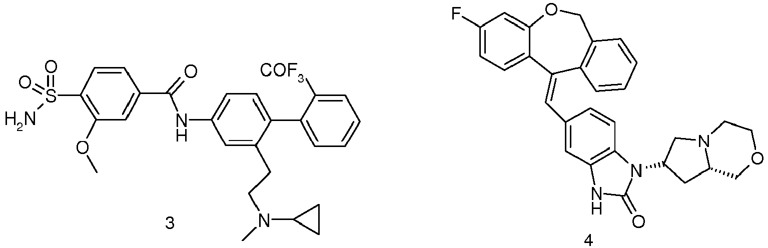

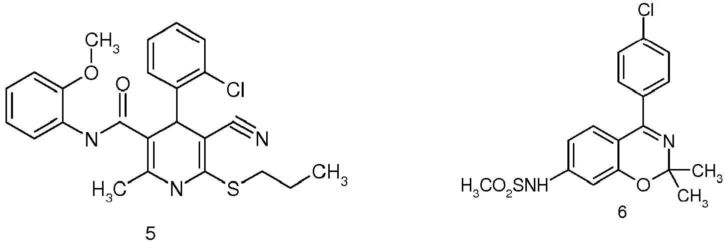

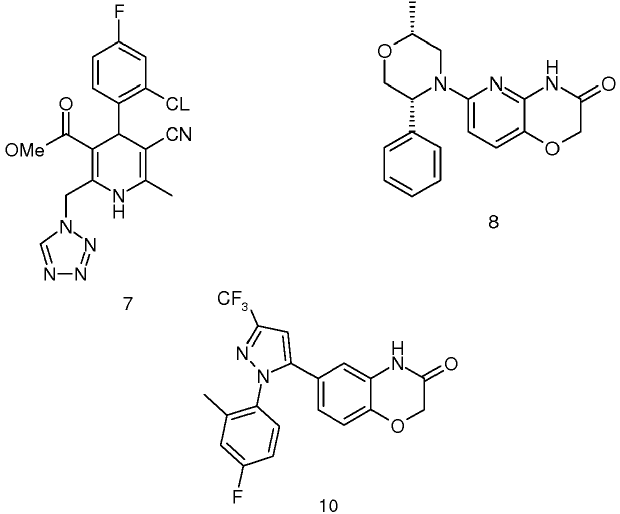

By a "therapeutically effective amount" is meant a sufficient amount of the MR antagonist for the treatment of CNV at a reasonable benefit/risk ratio applicable to any medical treatment. It will be understood that the total daily usage of the compounds and compositions of the present invention will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the MR antagonist for the symptomatic adjustment of the dosage to the subject to be treated. A medicament typically contains from about 0.01 mg to about 500 mg of the MR antagonist, preferably from 1 mg to about 100 mg of the MR antagonist. An effective amount of the drug is ordinarily supplied at a dosage level from 0.0002 mg/kg to about 20 mg/kg of body weight per day, especially from about 0.001 mg/kg to 7 mg/kg of body weight per day.

In some embodiments, the MR antagonist of the present invention is administered to the subject in combination with an anti-VEGF agent.

As used herein an "anti-VEGF agent" refers to a molecule that inhibits VEGF - mediated angiogenesis. For example, an anti-VEGF therapeutic may be an antibody to or other antagonist of VEGF. An "anti-VEGF antibody" is an antibody that binds to VEGF with sufficient affinity and specificity to be useful in a method of the invention. An anti-VEGF antibody will usually not bind to other VEGF homologues such as VEGF- B or VEGF-C, or other growth factors such as P1GF, PDGF or bFGF. A preferred anti- VEGF antibody is a monoclonal antibody that binds to the same epitope as the monoclonal anti-VEGF antibody A4.6.1 produced by hybridoma ATCC® HB 10709 and is a high-affinity anti-VEGF antibody. A "high-affinity anti-VEGF antibody" has at least 10-fold better affinity for VEGF than the monoclonal anti-VEGF antibody A4.6.1. Preferably the anti-VEGF antibody is a recombinant humanized anti-VEGF monoclonal antibody fragment generated according to WO 98/45331, including an antibody comprising the CDRs or the variable regions of Y0317. More preferably, anti-VEGF antibody is the antibody fragment known as ranibizumab (LUCENTIS ®). The anti-VEGF antibody ranibizumab is a humanized, affinity-matured anti-human VEGF Fab fragment. Ranibizumab is produced by standard recombinant technology methods in E. coli expression vector and bacterial fermentation. Ranibizumab is not glycosylated and has a molecular mass of -48,000 daltons. See WO98/45331 and U.S. 2003/0190317. Anti-VEGF agents include but are not limited to bevacizumab (rhuMab VEGF, Avastin®, Genentech, South San Francisco Calif.), ranibizumab (rhuFAb V2, Lucentis®, Genentech), pegaptanib (Macugen®, Eyetech Pharmaceuticals, New York N.Y.), sunitinib maleate (Sutent®, Pfizer, Groton Conn.). In some embodiments, the anti-VEGF agent is a dimeric fusion protein capable of binding VEGF with a high affinity composed of two receptor-Fc fusion protein consisting of the, principal ligand-binding portions of the human VEGFR1 or VEGFR2 receptor extracellular domains fused to the Fc portion of human IgGI (termed a "VEGF trap", aflibercept). Specifically, the VEGF trap consists of Ig domain 2 from VEGFR1, which is fused to Ig domain 3 from VEGFR2, which in turn is fused to the Fc domain of IgGI.

The method of the present invention is particularly suitable for the treatment of a subject refractory to an anti-VEGF treatment.

As used herein, the expression "subject refractory to an anti-VEGF treatment" applies to a subject who is non responder or insufficiently responder to the treatment with an anti-VEGF agent. By "nonresponder", it is meant that subject does not recover, ameliorate, or stabilize his condition with the anti-VEGF agent. For example, a subject refractory to anti-VEGF treatment is a subject which has been unsuccessfully treated with an anti-VEGF agent or a subject known to be unable to successfully respond to a treatment based on an anti-VEGF agent.

The MR antagonist is typically combined with pharmaceutically acceptable excipients, and optionally sustained-release matrices, such as biodegradable polymers, to form pharmaceutical compositions. The term "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In some embodiments, the MR antagonist of the present invention is administered to the subject by intraocular or periocular administration, either directly injected into the vitreous or in the peri-ocular spaces (sub conjunctival, sub tenon, peri bulbar, reto bular, intra scleral, supra choroidal).

In some embodiments, the MR antagonist of the present invention is administered to the subject in the form of polymeric implants and microspheres which can release drug loads over various time periods. These implants or microspheres, which when inserted into the subconjunctival (such as a sub-tenon) space or into the vitreous of an eye provide therapeutic levels of a MR antagonist, for extended periods of time (e.g., for about one week or more).

As used herein the term "microsphere" has its general meaning in the art and refers to a small diameter or dimension device or element that is structured, sized, or otherwise configured to be administered subconjunctivally (i.e. sub-tenon) or into the vitreous. Microspheres or microparticles includes particles, micro or nanospheres, small fragments, microparticles, nanoparticles, fine powders and the like comprising a biocompatible matrix encapsulating or incorporating a therapeutic agent. Microspheres are generally biocompatible with physiological conditions of an eye and do not cause significant adverse side effects. Microspheres administered intraocular can be used safely without disrupting vision of the eye. Microspheres have a maximum dimension, such as diameter or length, less than 1 mm. For example, microparticles can have a maximum dimension less than about 500 µm. Microspheres can also have a maximum dimension no greater than about 200 µm, or may have a maximum dimension from about 30 µm to about 50 µm, among other sizes. An "implant" is a drug delivery device which is considerably larger than a microsphere, and whereas a plurality (i.e. hundreds or thousands)) of microspheres are administered to treat an ocular condition (such as glaucoma) usually only one to at most six implants are administered for the same purpose.

Typically, the MR antagonist of the present implants is preferably from about 1% to 90% by weight of the microspheres. More preferably, the MR antagonist is from about 5% to about 30% by weight of the implant or microspheres. In a preferred embodiment, the MR antagonist comprises about 10% by weight of the microsphere (e.g., 5%-15%). In some embodiments, the MR antagonist comprises about 40% by weight of the microspheres.

Suitable polymeric materials or compositions for use in the implant or microspheres include those materials which are compatible that is biocompatible, with the eye so as to cause no substantial interference with the functioning or physiology of the eye. Such materials preferably are at least partially and more preferably substantially completely biodegradable. Biodegradable polymer" means a polymer or polymers which degrade in vivo, and wherein erosion of the polymer or polymers over time occurs concurrent with or subsequent to release of the therapeutic agent. The terms "biodegradable" and "bioerodible" are equivalent and are used interchangeably herein. A biodegradable polymer may be a homopolymer, a copolymer, or a polymer comprising more than two different polymeric units. Examples of useful polymeric materials include, without limitation, such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than about 5%, or less than about 1% of the polymeric material being cross-linked. For the most part, besides carbon and hydrogen, the polymers will include at least one of oxygen and nitrogen, advantageously oxygen. The oxygen may be present as oxy, e.g. hydroxy or ether, carbonyl, e.g. non-oxo-carbonyl, such as carboxylic acid ester, and the like. The nitrogen may be present as amide, cyano and amino. The polymers set forth in Heller, Biodegradable Polymers in Controlled Drug Delivery, In: CRC Critical Reviews in Therapeutic Drug Carrier Systems, Vol. 1, CRC Press, Boca Raton, Fla. 1987, pp 39-90, which describes encapsulation for controlled drug delivery, may find use in the present microspheres. Of particular interest are polymers of hydroxyaliphatic carboxylic acids, either homo- or copolymers, and polysaccharides. Included among the polyesters of interest are homo- or copolymers of D-lactic acid, L-lactic acid, racemic lactic acid, glycolic acid, caprolactone, and combinations thereof. Copolymers of glycolic and lactic acid are of particular interest, where the rate of biodegradation is controlled by the ratio of glycolic to lactic acid. The percent of each monomer in poly(lactic-co-glycolic)acid (PLGA) copolymer may be 0-100%, about 15-85%, about 25-75%, or about 35-65%. In some embodiments, 25/75 PLGA and/or 50/50 PLGA copolymers are used. The biodegradable ocular implants can also include additional hydrophilic or hydrophobic compounds that accelerate or retard release of the MR antagonist. Thus, implants or microspheres can be prepared where the center may be of one material and the surface may have one or more layers of the same or a different composition, where the layers may be cross-linked, or of a different molecular weight, different density or porosity, or the like. For example, where it is desirable to quickly release an initial bolus of drug, the center of the microsphere may be a polylactate coated with a polylactate-polyglycolate copolymer, so as to enhance the rate of initial degradation. Alternatively, the center may be polyvinyl alcohol coated with polylactate, so that upon degradation of the polylactate exterior the center would dissolve and be rapidly washed out of the eye.

The implant or microspheres may be of any particulate geometry including micro and nanospheres, micro and nanoparticles, spheres, powders, fragments and the like. The upper limit for the microsphere size will be determined by factors such as toleration for the implant, size limitations on insertion, desired rate of release, ease of handling, etc. Spheres may be in the range of about 0.5 µm to 4 mm in diameter, with comparable volumes for other shaped particles.

In addition to the therapeutic agent, the implants and microspheres disclosed herein may include or may be provided in drug delivery systems that include effective amounts of buffering agents, preservatives and the like. Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents advantageously present in amounts sufficient to maintain a pH of the system of between about 2 to about 9 and more preferably about 4 to about 8. As such the buffering agent may be as much as about 5% by weight of the total implant. Suitable water soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from about 0.001% to about 5% by weight and preferably about 0.01% to about 2% by weight. In at least one of the present microspheres, a benzylalkonium chloride preservative is provided in the implant. In addition to the MR antagonist included in the implants and microspheres disclosed herein, the microsphere may also include one or more additional ophthalmically acceptable therapeutic agents. For example, the microspheres may include one or more antihistamines, one or more antibiotics, one or more beta blockers, one or more steroids, one or more antineoplastic agents, one or more immunosuppressive agents, one or more antiviral agents, one or more antioxidant agents, and mixtures thereof. Alternatively, a single injection of implant or microspheres can include two or more microsphere batches each containing a different therapeutic agent or agents. Such a mixture of different implants and microspheres in included within the present invention.Additionally, release modulators such as those described in U.S. Pat. No. 5,869,079 may be included in the implants or microspheres. The amount of release modulator employed will be dependent on the desired release profile, the activity of the modulator, and on the release profile of the MR antagonist in the absence of modulator. Electrolytes such as sodium chloride and potassium chloride may also be included in the microspheres. Where the buffering agent or enhancer is hydrophilic, it may also act as a release accelerator. Hydrophilic additives act to increase the release rates through faster dissolution of the material surrounding the drug in the microspheres, which increases the surface area of the drug exposed, thereby increasing the rate of drug bioerosion. Similarly, a hydrophobic buffering agent or enhancer dissolves more slowly, slowing the exposure of drug, and thereby slowing the rate of drug bioerosion.

Typically, the microspheres of the present invention may be administered to subjects by administering an ophthalmically acceptable composition which comprises the microspheres to the subject. For example, microspheres may be provided in a liquid composition, a suspension, an emulsion, and the like, and administered by injection or implantation into the subconjunctival space of the eye.A syringe apparatus including an appropriately sized needle, for example, a 22 gauge needle, a 27 gauge needle or a 30 gauge needle, can be effectively used to inject the microspheres into the subconjunctival space or into the vitreous.

The invention will be further illustrated by the following figures and examples. However, these examples and figures should not be interpreted in any way as limiting the scope of the present invention.

### FIGURES:

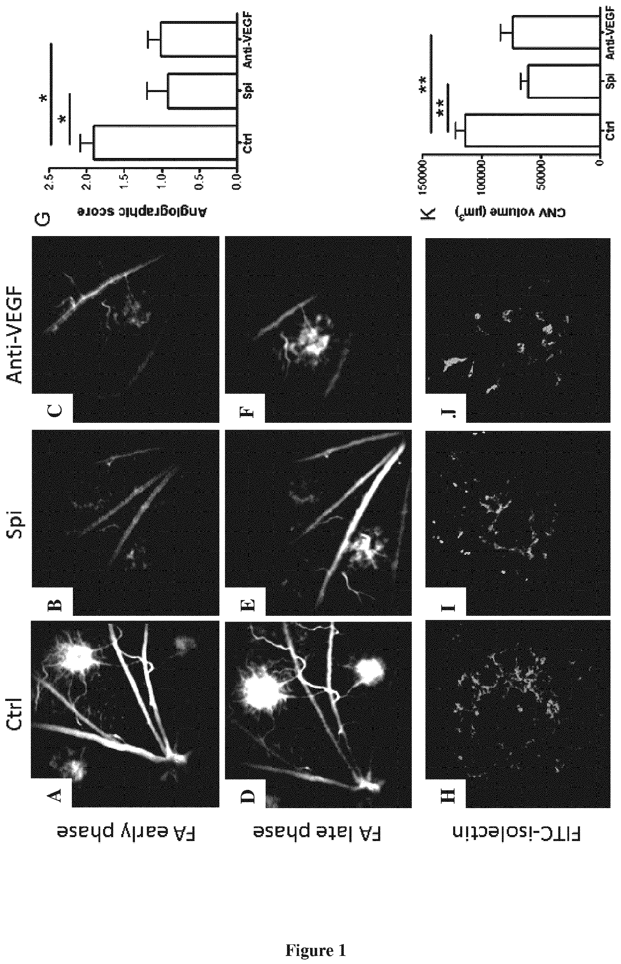

**Figure 1****. Subcutaneous administration of spironolactone limits choroidal neovascularisation (CNV) induced by laser in rat eyes.**
   Representative early (A-C) and late phase (D-F) fluorescein angiograms (FA) performed at the peak of CNV (ie. 14 days after laser induction) highlight reduced vascular leakage in the spironolactone-treated group (spi) compared to the laser control group. Intravitreous anti-VEGF treatment serves as positive control. Grading of vascular leakage on FA (G) shows significant decrease in angiographic score with spironolactone treatment (-45%) that is comparable to anti-VEGF group (-37%). Results are expressed as mean angiographic score/bum/rat ± SEM. In each of both eyes, 6 laser burns were made, n= 11 rats for control group, 6 for spironolactone group, and 5 for anti-VEGF group. Kruskal-Wallis test was used followed by Dunn's comparison. *, P<0.05. Combined treatment with intravitreous anti-VEGF + subcutaneous spironolactone shows significant additive effect with -84% of leakage as compared to control (nor shown).
   CNV in retinal pigment epithelium/choroid flatmounts were labelled with FITC-GSL I-Isolectin B4. Spironolactone-treated group (I) shows reduced CNV size compared to the laser control (H). Anti-VEGF treatment (J) is the positive control group. Quantification of the size of CNV (K) show significant decrease in CNV volume in spironolactone and anti-VEGF groups compared to laser control. Results are expressed as mean CNV volume/burn/rat ± SEM. Six laser burns per rat eye, n=11 rats for control group, 6 for spironolactone group, and 7 for anti-VEGF group. Kruskal-Wallis test followed by Dunn's multiple comparison was used. **, P<0.01.

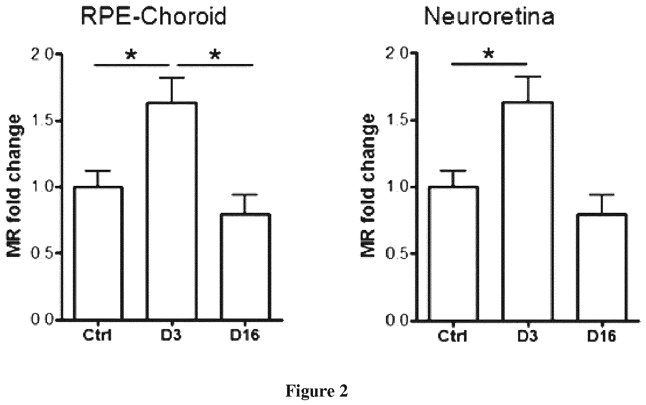

**Figure 2****. MR expression in rat retina tissues at different time point after laser induction.** At day 3 after laser (peak of macrophage infiltration), MR is up-regulated in both retinal pigment epithelium (RPE)-choroid complex (left) and neuroretina (right), compared to control normal rat eyes (ctrl, without laser treatment). At day 16, peak of neovascularisation, MR expression in RPE-Choroid (left) and neuroretina (right) was found to decline to the same level of controls. Results are expressed as mean ± SEM. n=6 rats for control, 5 rats for D3 and 4 rats for D16. Kruskal-Wallis test was used followed by Dunn's comparison. *, P<0.05.

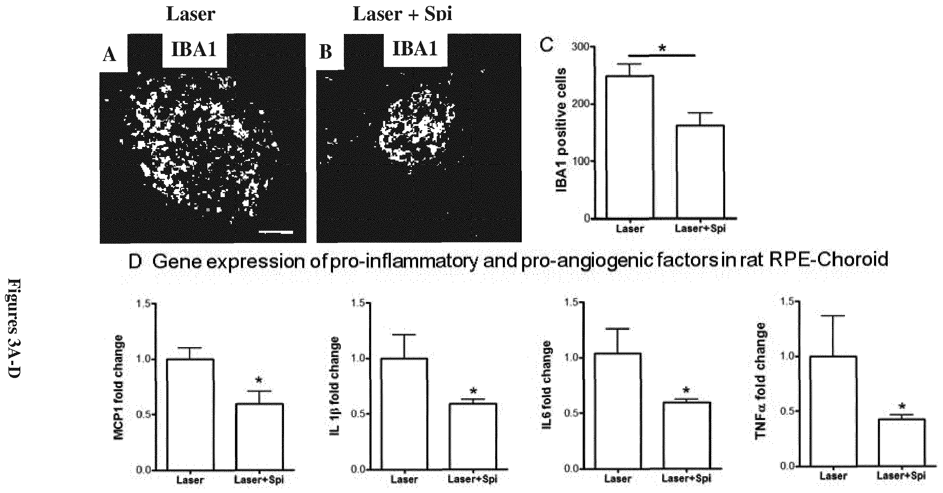

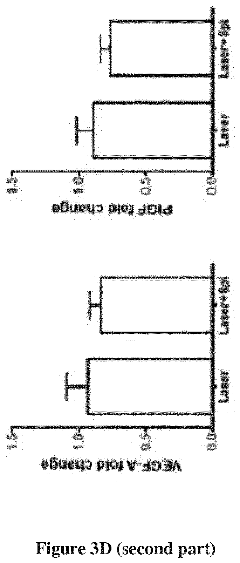

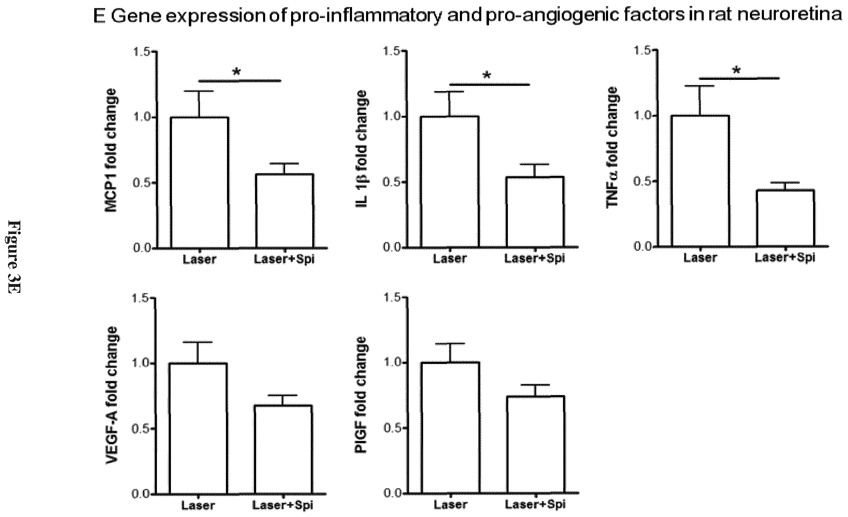

**Figure 3****. Spironolactone inhibits macrophage/microglial recruitment and down-regulates pro-inflammatory gene expression in rat retinas 3 days after laser.**
   The peak of macrophage/microglial infiltration/activation occurs at day 3 after laser. We labelled these cells with IBA1 on retinal pigment epithelium (RPE)-choroid flatmounts. Round activated IBA1 positive cells were counted in the areas with laser burns. Spironolactone (Spi) decreases IBA1 positive cells in the burns (B) compared to the non-treated eyes (A). Cell quantification shows significant fewer activated macrophage/microglia observed in spironolactone treated eyes than in laser control group (C). Bars in A and B: 100 µm. Results are expressed as mean IBA1 positive cells/burn/rat ± SEM. Six laser burns per rat eye, n=7 rats for laser group and 4 rats for laser+Spi group. Mann Whitney U test was used, * P<0.05.
   Quantitative PCR at day 3 show that spironolactone down-regulates the laser-induced gene expression of MCP1, IL1β, IL6 and TNFα in the RPE-choroid complex (D), and MCP1, IL1β and TNFα in the neuroretina (E). However, the expression of pro-angiogenic factors, VEGF-A and PlGF, is not regulated at RNA level (D and E) showing that spironolactone effect is independent from VEGF and PlGF. Results are expressed as mean ± SEM, n=5 rats for laser group and 6 rats for spironolactone group. Mann-Whitney U test was used. * P<0.05.

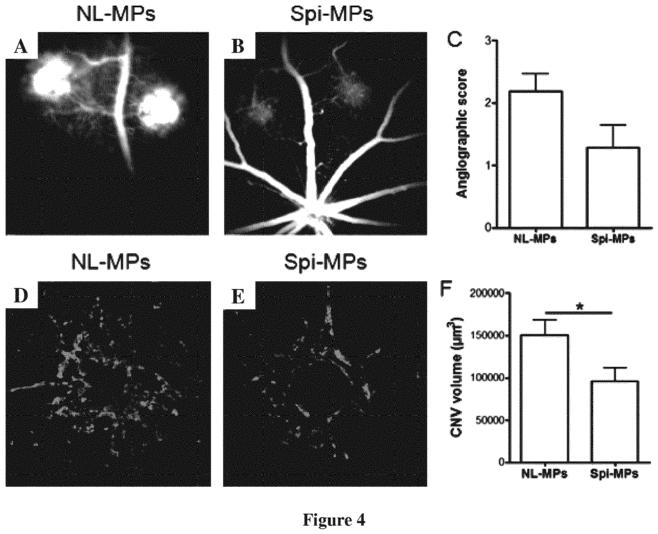

**Figure 4****. Intravitreous poly(lactide-co-glycolide) microspheres releasing spironolactone limit laser-induced CNV in rat eyes.**
   Fluorescein angiograms performed at day 14 after laser show reduced fluorescein intensity in eyes injected with spironolactone-loaded microspheres (Spi-MPs, B) compared to eyes injected with non-loaded microspheres (NL-MPs, A). We observed a tendency to decrease in angiographic score in Spi-MPs group compared to NL-MPs group (C), however, the difference is not significant. Results are expressed as mean angiographic score/burn/rat ± SEM. In each of both eyes, 6 laser burns were made, n= 5 rats in NL-MPs group and 7 rats in Spi-MPs group. Mann Whitney U test was used for statistic analysis.
   CNV in retinal pigment epithelium/choroid flatmounts were labelled with FITC-GSL I-Isolectin B4. We observed a decrease in CNV staining in Spi-MPs group (E) compared to NL-MPs group (D). Quantification of CNV on z-stack confocal images show that CNV volume significantly declines in eyes injected with Spi-MPs compared to NL-MPs injected eyes. Results are expressed as mean CNV volume/burn/rat ± SEM. Six laser burns per rat eye, n=8 rats each group. Mann Whitney U test was used, * P<0.05.

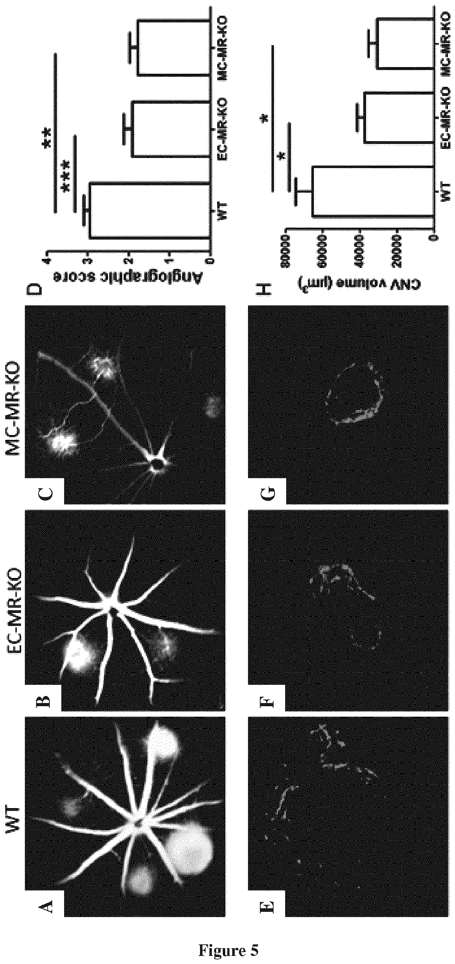

**Figure 5****. Laser-induced CNV in vascular endothelium-specific MR knock-out mice (EC-MR-KO) and in monocyte-specific MR knock-out mice (MC-MR-KO).**
   Fluorescein angiography (FA) was performed at day 10 after laser, corresponding to the peak of CNV development in mice (A-D). At day 12, after intravenous perfusion with FITC-Dextran, eyes were enucleated. Perfused CNV was observed in retinal pigment epithelium-choroid flatmounts (E-H).
   FA shows reduced fluorescein leakage in the eyes of both tissue-specific MR knock-out mice (B and C), compared to wild-type mice (C57BL/6) (WT, A), which is confirmed by angiographic scoring (D). Results are expressed as mean score/laser burn ± SEM. Four laser burns per eye, n=68 burns for WT, 60 for EC-MR-KO and 16 for MC-MR-KO mice. Kruskal-Wallis test followed by Dunn's multiple comparison was used, ** P<0.01, *** P<0.001.
   FITC-GSL I-Isolectin B4 labelling shows smaller CNV developed in eyes of EC-MR-KO (F) and MC-MR-KO mice (G) than in WT mice (E). Quantification of CNV on z-stack confocal images finds that CNV size is decreased in both MR knock-out mice compared to WT (H). Results are expressed as mean CNV volume/laser burn ± SEM. Four burns per eye, n=80 burns for WT, 68 for EC-MR-KO and 24 for MC-MR-KO mice. Kruskal-Wallis test followed by Dunn's multiple comparison was used, * P<0.05.

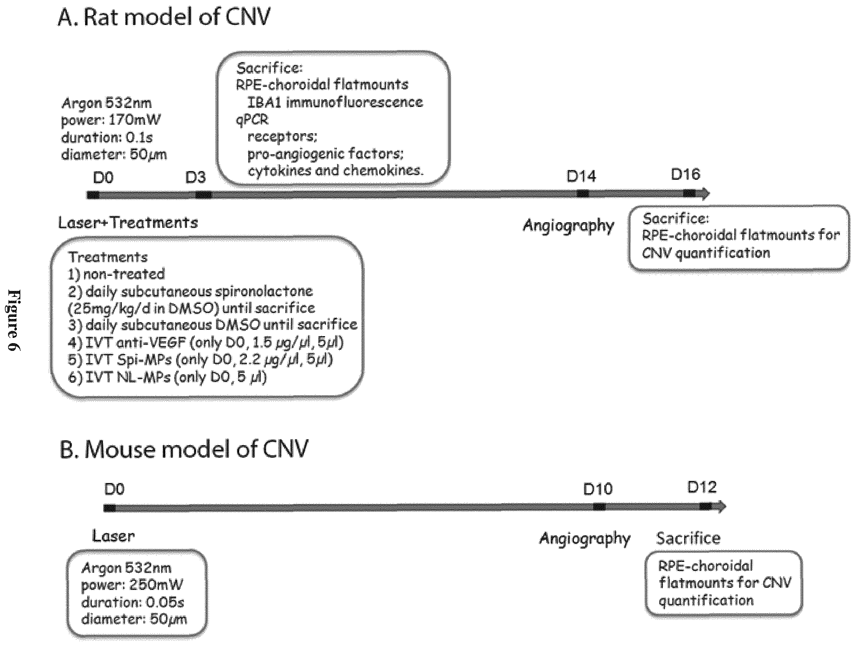

**Figure 6****. Experimental designs of rat (A) and mouse (B) models of laser-induced CNV.**

### EXAMPLE:

### Materials & Methods

### Animals

All experiments were performed in accordance with the European Communities Council Directive 86/609/EEC and approved by local ethical committees (Ce5/2012/113). Six to eight-week old male Brown Norway rats from Janvier breeding Center (Le Genest-Saint-Isle, France) were used to create rat model of CNV. Vascular endothelial cell-specific MR knock-out mice (EC-MR-KO) and monocyte/macrophage-specific MR knock-out mice (MC-MR-KO) were generated in C57BL/6 genetic background mice and were obtained from team of Dr F. Jaisser (UMRS 1138, team 1). EC-MR-KO mice were created by mating transgenic mice expressing Cre recombinase in endothelial cells with mice harbouring MR-floxed alleles (Berger S et al. Loss of limbic mineralocorticoid receptor impairs behavioural plasticity. PNAS USA 2006). MC-MR-KO mice were obtained by mating mice expressing Cre recombinase in monocytes/macrophages (Clausen BE et al. Conditional gene targeting in macrophages and granulocytes using LysMcre mice. Trangenic Research 1999) with mice harbouring MR-floxed alleles. Animals were kept in pathogen-free conditions with food, water and litter and housed in a 12-hour light/12-hour dark cycle. Anesthesia was induced by intramuscular ketamine (40 mg/kg for rats, 50 mg/kg for mice) and xylazine (4 mg/kg for rats, 10 mg/kg for mice). Animals were sacrificed by carbon dioxide inhalation or cervical dislocation.

### Experimental designs

The experimental designs of rat and mouse models of laser-induced CNV are detailed in the figure 6. The treatments given in rat CNV model were considered as prevention.

### Laser coagulation

After anesthesia and dilation of the pupils, coverslips were positioned on the cornea as a contact glass. For rats, six to eight burns were performed 2 to 3-disk diameters away from the papillae with an Argon laser (532 nm) mounted on a slit lamp (170 mW, 0.1 second and 50 µm). For mice, four laser burns were induced at the 3, 6, 9 and 12 o'clock positions around the optic disc (250 mW, 0.05 second and 50 µm). The presence of a bubble witnessed the rupture of Bruch's membrane and confirmed the success of the laser impact.

### Treatments

Treatments were introduced only in rat model of CNV. After laser photocoagulation, rats were divided into 6 groups: 1) non-treated; 2) daily subcutaneous injection of spironolactone diluted in oliver oil and DMSO (25 mg/kg/day) until sacrifice; 3) daily subcutaneous injection of vehicle (oliver oil and DMSO) until sacrifice; 4) intravitreous injection (IVT) of anti-rat VEGF (1.5 µg/µl, 5 µl, RnD system, Lille, France) only at day 0 (Couturier A et al. Anti-vascular endothelial growth factor acts on retinal microglia/macrophage activation in a rat model of ocular inflammation. Mol Vis 2014;20:908-20); 5) IVT of spironolactone-loaded PLGA MPs only at day 0 (2.2 µg/µl, 5 µl, obtained from team of Pr R. Herrero-Vanrell, Complutense University of Madrid). Considering the *ex vivo* release profile of Spi-MPs, the daily dose of spironolactone released in the vitreous is estimated at 18 µM; 6) IVT of blank PLGA MPs (5 µl) only at day 0.

### Fluorescein angiography (FA)

FA was performed 14 days (in rats) or 10 days (in mice) after laser induction. Pupils dilated, fluorescein (0.2 mL (rat) or 0.1 mL (mouse) of 10% fluorescein in saline) was injected intravenously in the tail. Early and late phase angiograms were recorded 1-3 and 5-7 min respectively after fluorescein injection. For each laser-induced lesion, fluorescein leakage was graded qualitatively by evaluating the increase in size/intensity of dye between the early and late phase. Angiographic scores were established by two masked observers according to the following criteria: grade 0 indicates no hyperfluorescence; grade 1 indicates a slight hyperfluorescence with no increase in intensity nor in size; grade 2 indicates a hyperfluorescence increasing in intensity but not in size; grade 3 indicates a hyperfluorescence increasing both in intensity and size. An additional grade 4 was given when the hyperfluorescence size increase is more than 2 diameter of initial laser burn.

### RPE-choroidal flatmounts and CNV quantifications

Two days after FA examination (time necessary for fluorescein elimination), eyes were enucleated, fixed in 4% paraformaldehyde for 15 min at room temperature and sectioned at the limbus; the cornea and lens were discarded. Retina was separated from RPE-choroid complex. Eight radial incisions were made on RPE-choroid which was then flatmounted and post-fixed with acetone for 15 min at -20°C. After washing with 0.1% Triton x100 in PBS, FITC-GSL I-Isolectin B4 (1:200, Vector) was applied over night at -4°C. After washing with PBS, RPE-choroid was flatmounted and observed with a confocal microscope (Zeiss LSM710, Le Pecq, France). Images of the CNV were captured with a digital video camera coupled to a computer system. Horizontal optical sections (1 µm interval) were obtained from the surface of the CNV. The deepest focal plane in which the surrounding choroidal vascular network connecting to the lesion could be identified was judged to be the floor of the CNV lesion. The area of CNV-related fluorescence on each horizontal section was measured using ImageJ software. The summation of the whole fluorescent area on z-stack images from the top to the bottom of the CNV was used as an index for the CNV volume.

Mice were perfused with FITC-dextran (molecular weight 2,000,000, Sigma-Aldrich, St-Quentin Fallavier, France) before enucleation. After RPE-choroidal flatmounting, FITC-dextran perfused CNV was examined and analyzed as previously described.

### Immunofluorescence on RPE-choroidal flatmounts

Three days after laser induction (peak of macrophage infiltration), rat RPE-choroidal flatmounts were also prepared for immunofluorescence. Rabbit polyclonal anti-IBAl (1:400, Wako, Neuss, Germany) was applied over night at -4°C. After washing with 0.1% Triton x100/PBS, flatmounts were incubated with Alexa Fluo® 594 goat anti-rabbit IgG (1:200, Molecular Probes, Leiden, The Netherlands), the nuclei were counterstained with DAPI. IBA1 positive macrophage/microglial cells were counted on stained laser lesions. The average cell number/per impact/rat eye was calculated.

### Quantitative PCR

Three days after laser induction, rat neuroretinas and RPE-choroid-sclera complexes were carefully dissected from enuleated eyes and snap-frozen in liquid nitrogen and stored at - 80°C until use. Total RNA was isolated from tissues using RNeasy Plus Mini Kit (Qiagen, Courtaboeuf, France) according to the manufacturer's instructions. First-strand complementary DNA was synthesized from total mRNA using random primers (Life Technologies, Cergy Pontoise, France) and SuperScript II reverse transcriptase (Life Technologies). Transcript levels of MR, MCP1, IL6, IL1β, TNFα, VEGF-A and PlGF were analyzed by quantitative real-time PCR performed in 7500 Real-Time PCR System (Applied Biosystems, Foster City, CA, USA) with either SYBR Green or TaqMan detection.

As preliminary results showed no difference in CNV volume and angiographic score between non-treated laser group and vehicle-treated laser group, we pooled the results of these two groups as controls for subcutaneous spironolactone-treated laser group.

### Statistics

Results are expressed as mean ± SEM. Statistical analyses were carried out using GraphPad Prism 5 for Windows (GraphPad Software Inc., San Diego, CA, USA). Nonparametric Kruskal-Wallis and Mann-Whitney tests were used to compare continuous data when appropriate. P-values of 0.05 or less were considered significant.

### Results

Our results show that systemic administration of MR antagonist, spironolactone, limits the CNV development, effect comparable to that of anti-VEGF injected intravitreously in the rat eyes (Figure 1).

As MR is up-regulated in early phase of the disease (Figure 2), and MR antagonism inhibits the accumulation of activated macrophage/microglial cells in the laser burned areas (Figure 3A-C), MR over-activation may play a role in the early inflammation induced by laser through macrophage/microglial activation and finally contribute to the CNV development. At day 3, laser-induced expression of cytokines (TNFα, Il1β, IL6) and chemokines (MCP1), inflammatory components known to be implicated in the CNV formation (Lavalette Sophie et al. Il1β inhibition prevents CNV and does not exacerbate photoreceptor degeneration. Am J Pathol 2011; Raoul W, CCL2/CCR2 and CX3CL1/CX3CR1 chemokine axes and their possible involvement in age-related macular degeneration. J Neuroinflammation 2011; Kramer M et al. MCP1 in the aqueous humour of subjects with age-related macular degeneration. Clin Experiment Ophthalmol 2012; Miao H et al. Inflammatory cytokines in aqueous humor of subjects with CNV. Mol Vis 2012), is abrogated by spironolactone (Figure 3D and E), however the expression of pro-angiogenic factors (VEGF and PGF) is not modified , suggesting that MR antagonist acts through pathways other than that of VEGF to prevent CNV development.

To limit eventual systemic side effects associated with long-term use of MR antagonist, microspheres controlled releasing spironolactone were developed and injected intravitreously in the rat eyes. They also show beneficial effect to prevent CNV (Figure 4).

Macrophages/microglia and vascular endothelial cells (EC) are critical for the pathogenesis of CNV, as they express VEGF and cytokines and chemokines which in turn regulate monocyte and endothelial cell recruitment (Grossniklaus et al. Macrophage and retinal pigment epithelium expression of angiogenic cytokines in CNV. Mol Vis 2002; Kvanta et al. Subfoveal fibrovascular membranes in AMD express VEGF. IOVS 1996). Macrophages and choroidal EC also express MR. In order to evaluate the role of macrophage MR and endothelial MR in CNV formation, we performed laser coagulation using EC-specific MR knockout mice and monocyte/macrophage-specific MR knockout mice. Both mice show reduced CNV formation (Figure 5), suggesting EC MR and macrophage MR contribute to CNV development.

### REFERENCES:

Throughout this application, various references describe the state of the art to which this invention pertains.

## Claims

1. A mineralocorticoid receptor antagonist for use in the treatment of choroidal neovascularisation in a subject in need thereof wherein the subject is refractory to an anti-VEGF treatment.

2. The mineralocorticoid receptor antagonist for use according to claim 1 wherein the subject suffers from a disease selected from the group consisting of age-related macular degeneration, myopic choroidal neovascularization, idiopathic choroidal neovascularization, polypoidal chorioretinopathy associated or not to central serous chorioretinoapthy and some inflammatory conditions such as such uveitis posterior, traumatic choroidal rupture, angioid streaks, and ocular histoplasmosis syndrome.

3. The mineralocorticoid receptor antagonist for use according to claim 1 wherein the choroidal neovascularisation is secondary to age-related macular degeneration.

4. The mineralocorticoid receptor antagonist for use according to claim 1 wherein the choroidal neovascularisation is secondary to pathological myopia.

5. The mineralocorticoid receptor antagonist for use according to claim 1 wherein the choroidal neovascularisation is an idiopathic choroidal neovascularisation.

6. The mineralocorticoid receptor antagonist for use according to claim 1 wherein the subject suffers from polypoidal choroidal vasculopathy.

7. The mineralocorticoid receptor antagonist for use according to claim 1 wherein the MR antagonist is an epoxy-steroidal mineralocorticoid receptor antagonist or a non-epoxy-steroidal mineralocorticoid receptor antagonist.

8. The mineralocorticoid receptor antagonist for use according to claim 1 wherein the MR antagonist is administered to the subject in combination with an anti-VEGF agent.

9. The mineralocorticoid receptor antagonist for use according to claim 1 wherein the MR antagonist is administered to the subject by intraocular or periocular administration, either directly injected into the vitreous or in the peri-ocular spaces such as sub conjunctival, sub tenon, peri bulbar, reto bular, intra scleral, supra choroidal.

10. The mineralocorticoid receptor antagonist for use according to claim 1 wherein the MR antagonist is administered to the subject in the form of microspheres that are injected into the subconjunctival space or into the vitreous.

## Patentansprüche

1. Mineralkortikoid-Rezeptorantagonist zur Verwendung bei der Behandlung von choroidaler Neovaskularisation bei einem diesbezüglich bedürftigen Patienten, wobei der Patient resistent gegen eine Anti-VEGF-Behandlung ist.

2. Mineralkortikoid-Rezeptorantagonist zur Verwendung nach Anspruch 1, wobei der Patient an einer Krankheit leidet, die ausgewählt ist aus der Gruppe bestehend aus altersbedingter Makuladegeneration, myoper choroidaler Neovaskularisation, idiopathischer choroidaler Neovaskularisation, polypoidaler Chorioretinopathie, die mit der zentralen serösen Chorioretinoapthie assoziiert ist oder nicht, und einigen entzündlichen Zuständen, wie z.B. Uveitis posterior, traumatische Aderhautruptur, angioide Streifen und okuläres Histoplasmose-Syndrom.

3. Mineralkortikoid-Rezeptorantagonist zur Verwendung nach Anspruch 1, wobei die choroidale Neovaskularisation sekundär zu der altersbedingten Makuladegeneration ist.

4. Mineralkortikoid-Rezeptorantagonist zur Verwendung nach Anspruch 1, wobei die choroidale Neovaskularisation sekundär zu der pathologischen Myopie ist.

5. Mineralkortikoid-Rezeptorantagonist zur Verwendung nach Anspruch 1, wobei die choroidale Neovaskularisation sekundär zu der choroidalen Neovaskularisation ist.

6. Mineralkortikoid-Rezeptorantagonist zur Verwendung nach Anspruch 1, wobei der Patient an einer polypoidalen choroidalen Vaskulopathie leidet.

7. Mineralkortikoid-Rezeptorantagonist zur Verwendung nach Anspruch 1, wobei der MR-Antagonist ein epoxysteroidaler Mineralkortikoid-Rezeptorantagonist oder ein nicht-epoxysteroidaler Mineralkortikoid-Rezeptorantagonist ist.

8. Mineralkortikoid-Rezeptorantagonist zur Verwendung nach Anspruch 1, wobei der MR-Antagonist dem Patienten in Kombination mit einem Anti-VEGF-Mittel verabreicht wird.

9. Mineralkortikoid-Rezeptorantagonist zur Verwendung nach Anspruch 1, wobei der MR-Antagonist dem Patienten durch intraokuläre oder periokuläre Verabreichung verabreicht wird, entweder direkt in den Glaskörper oder in die periokulären Räume, wie z.B. subkonjunktival, sub-Tenon, peribulbär, retrobulbär, intraskleral, suprachoroidal.

10. Mineralkortikoid-Rezeptorantagonist zur Verwendung nach Anspruch 1, wobei der MR-Antagonist dem Patienten in Form von Mikrosphären verabreicht wird, die in den subkonjunktivalen Raum oder in den Glaskörper injiziert werden.

## Revendications

1. Antagoniste du récepteur minéralocorticoïde destiné à être utilisé dans le traitement de la néovascularisation choroïdienne chez un patient en ayant besoin, dans lequel le sujet est réfractaire à un traitement anti-VEGF.

2. Antagoniste du récepteur minéralocorticoïde destiné à être utilisé selon la revendication 1, dans lequel le sujet souffre d'une maladie sélectionnée dans le groupe constitué par la dégénérescence maculaire liée à l'âge, la néovascularisation choroïdienne myopique, la néovascularisation choroïdienne idiopathique, la choriorétinopathie polypoïdale associée, ou non, à la choriorétinopathie sérieuse centrale et à certains états inflammatoires tels qu'une uvéite postérieure, une rupture choroïdienne traumatique, des stries angioïdes et le syndrome d'histoplasmose oculaire.

3. Antagoniste du récepteur minéralocorticoïde destiné à être utilisé selon la revendication 1, dans lequel la néovascularisation choroïdienne est secondaire à une dégénérescence maculaire liée à l'âge.

4. Antagoniste du récepteur minéralocorticoïde destiné à être utilisé selon la revendication 1, dans lequel la néovascularisation choroïdienne est secondaire à une myopie pathologique.

5. Antagoniste du récepteur minéralocorticoïde destiné à être utilisé selon la revendication 1, dans lequel la néovascularisation choroïdienne est une néovascularisation choroïdienne idiopathique.

6. Antagoniste du récepteur minéralocorticoïde destiné à être utilisé selon la revendication 1, dans lequel le sujet souffre d'une vasculopathie polypoïdale choroïdienne.

7. Antagoniste du récepteur minéralocorticoïde destiné à être utilisé selon la revendication 1, dans lequel l'antagoniste du récepteur minéralocorticoïde est un antagoniste époxy-stéroïdien du récepteur minéralocorticoïde ou un antagoniste non époxy-stéroïdien du récepteur minéralocorticoïde

8. Antagoniste du récepteur minéralocorticoïde destiné à être utilisé selon la revendication 1, dans lequel l'antagoniste du récepteur minéralocorticoïde est administré au sujet en combinaison avec un agent anti-VEGF.

9. Antagoniste du récepteur minéralocorticoïde destiné à être utilisé selon la revendication 1, dans lequel l'antagoniste du récepteur minéralocorticoïde est administré au sujet au moyen d'une administration intraoculaire ou périoculaire, injecté directement soit dans le vitré, soit dans les espaces périoculaires tels que les espaces sous-conjonctival, sous ténonien, péribulbaire, rétrobulbaire, intrascléral, supra choroïdien.

10. Antagoniste du récepteur minéralocorticoïde destiné à être utilisé selon la revendication 1, dans lequel l'antagoniste du récepteur minéralocorticoïde est administré au sujet sous la forme de microsphères qui sont injectées dans l'espace sous-conjonctival ou dans le vitré.
